(19) Europäisches Patentamt
European Patent Office
Office européen des brevets

(11) **EP 3 067 809 A1**

(12) **EUROPEAN PATENT APPLICATION**

(43) Date of publication:
14.09.2016 Bulletin 2016/37

(51) Int Cl.:
*G06F 19/10* [(2011.01)]    *G06N 3/12* [(2006.01)]
*G11C 13/00* [(2006.01)]

(21) Application number: 15305381.4

(22) Date of filing: 13.03.2015

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(71) Applicant: Thomson Licensing
92130 Issy-les-Moulineaux (FR)

(72) Inventors:
• Blawat, Meinolf
30659 Hannover (DE)
• Bolot, Jean
LOS ALTOS, CA California 84024 (US)
• Eriksson, Brian
San Jose, CA 95134 (US)

• Lasserre, Sebastien
35235 Thorigné Fouillard (FR)
• Crovella, Mark
Wayland, MA 01778 (US)
• May, Martin
75015 Paris (FR)
• Gaedke, Klaus
30659 Hannover (DE)
• Wittenburg, Jens Peter
30916 Isernhagen (DE)
• Diot, Christophe
750005 Paris (FR)
• Le Scouarnec, Nicolas
35340 Liffre (FR)

(74) Representative: Huchet, Anne et al
Technicolor
1, rue Jeanne d'Arc
92443 Issy-les-Moulineaux (FR)

(54) **Method and apparatus for storing and selectively retrieving data encoded in nucleic acid molecules**

(57) Methods and apparatuses for storing multiple data sets in nucleic acid molecules (70, 80) and for selective retrieval (90, 100), as well as corresponding nucleic acid molecules (148, 158), are presented. The structure of the synthesized nucleic acid molecules allows selective amplification and sequencing for selective retrieval of encoded data sets. The data sets are partitioned (72) into a plurality of data set portions and said data set portions into data snippets. A plurality of nucleic acid molecules is synthesized (73) which comprise a plurality of primer segments and a data storage segment wherein a sequence of nucleotides is arranged to encode a corresponding data snippet of a corresponding one of the data set portions of a corresponding one of the data sets, wherein a first primer segment comprises an identifier of the corresponding data set and a second primer segment comprises an identifier of the corresponding data set portion.

Fig. 14

**Description**

**FIELD**

Methods and apparatuses for storing data in nucleic acid molecules and retrieving selected portions of the data are presented. In particular, the present disclosure relates to a method and apparatus for storing a plurality of data sets in nucleic acid molecules, to a nucleic acid molecule, and to a corresponding method and apparatus for retrieval of selected data from nucleic acid molecules.

**BACKGROUND**

A nucleic acid is a polymeric macromolecule and consists of a sequence of monomers known as nucleotides. A nucleotide consists of a sugar component, a phosphate group and a nitrogenous base or nucleobase. Nucleic acid molecules where the sugar component of the nucleotides is deoxyribose are DNA (deoxyribonucleic acid) molecules, whereas nucleic acid molecules where the sugar component of the nucleotides is ribose are referred to as RNA (ribonucleic acid) molecules. DNA and RNA are the biochemical storage molecules of genetic information appearing in living organisms.

Nucleic acid molecules are assembled as chains or strands of nucleotides. For instance, RNA molecules are single helical nucleic strands, while DNA double helix molecules are formed out of two antiparallel nucleic strands.

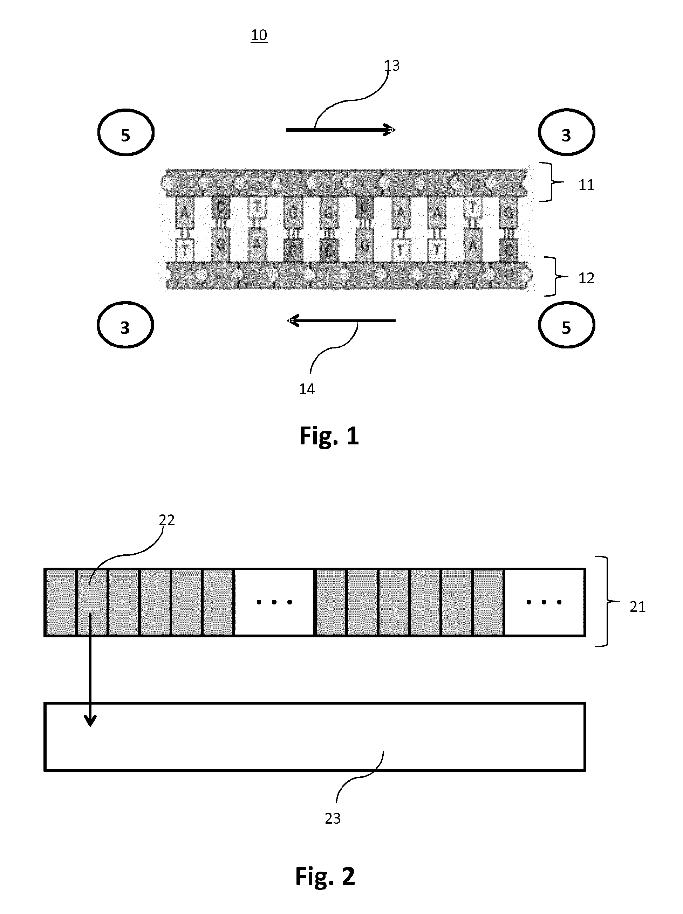

As schematically illustrated in Fig. 1, DNA molecules consist of two strands consisting of a series of different molecules bonded together, similar to the structure of a common ladder. The schematically shown fragment of a DNA molecule 10 contains two strands 11, 12 which may be regarded as the ladder-bars while the different molecules bonded together may be regarded as the ladder-steps.

DNA strands are built from four different nucleotides identified by their respective nucleobases or nitrogenous bases, namely, Adenine, Thymine, Cytosine and Guanine, which are denoted shortly as A, T, C and G, respectively, as indicated in Fig. 1. As another example, RNA strands also consist of four different nucleotides identified by their respective nucleobases, namely, Adenine, Uracil, Cytosine and Guanine, which are denoted shortly as A, U, C and G, respectively.

Each of the DNA ladder-steps is formed by pairs of the four nucleotides while only two combinations of such base pairs occur. Guanine goes together with Cytosine (G-C), while Adenine connects with Thymine (A-T). In this context, A and T, as well as C and G, are called complementary. Guanine, Adenine, Thymine, and Cytosine are the nucleobases of the nucleotides, while their connections are addressed as base pairs. The example of a DNA molecule 10 shown in Fig. 1 is a series of nucleotides bonded to the two strands 11, 12. Due to biochemical reasons, DNA strands have a predominant direction how they are read or biochemically interpreted. As shown in Fig. 1, this predominant direction is commonly indicated with '5' at the starting edge and '3' at the ending edge. Further, the predominant direction of strand 11 is indicated by arrow 13, whereas the predominant direction of strand 12 is indicated by arrow 14.

The predominant direction of DNA strands allows assigning logically to each base pair of a strand a piece of information. For example, logical values can be assigned to the four nucleotides, identified by their nucleobases, as follows: 0 to G, 1 to A, 2 to T, and 3 to C. Since arbitrary series of nucleotides can be synthesized any digital information can be stored in DNA strands. The data can be any kind of sequential digital data, e.g., sequences of quaternary code symbols, corresponding to digitally, for example binary, encoded information, such as textual, image, audio or video data. For storing data in synthesized, i.e. artificially created, nucleic acid molecules, usually comparably short DNA or RNA molecules, i.e. oligonucleotides (short: oligos) are generated. Due to the limited oligo length, the data is usually distributed to multiple oligos.

With these nucleic acid molecules, i.e., oligos, a data storage system can be realized. The synthesized nucleic acid molecules carry the information encoded by the succession of the different nucleotides forming the nucleic acid molecules. Each of the synthesized nucleic acid molecules consists of a sequence or chain of nucleotides generated by a biological, biochemical and/or biophysical process using a nucleic acid synthesizer. For example, in a DNA storage system, short DNA oligos are synthesized.

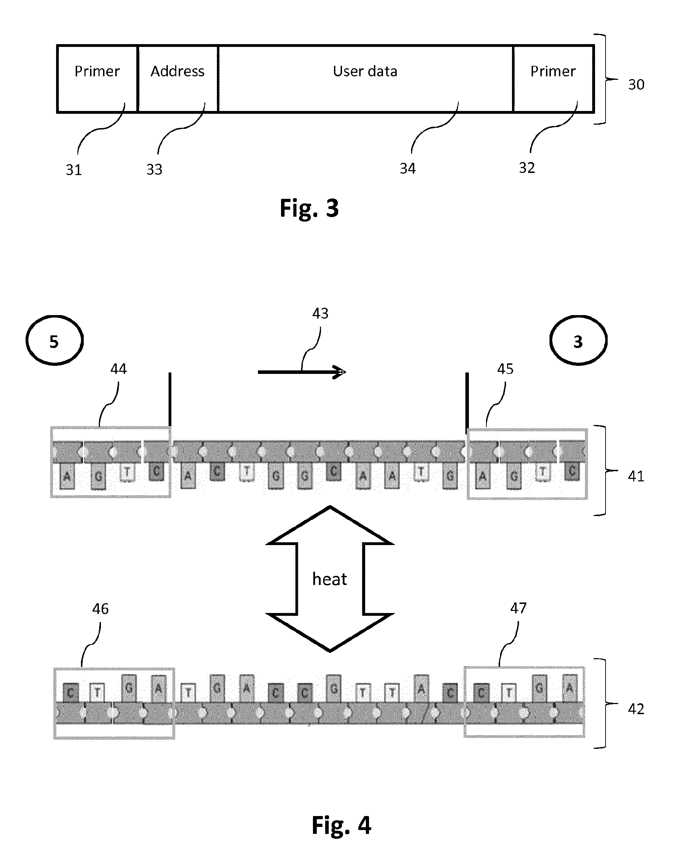

Synthesizers can produce oligos with a low error rate only of a certain length. For lengths that go beyond, the error rates increase. For example, some synthesizers produce nucleic acid molecules having a length of up to 350 nucleotides. The possible lengths depend on the working mechanism of the deployed synthesizer. As schematically illustrated in Fig. 2, data to be stored 21 is cut into snippets, while each snippet 22 is logically assigned to a nucleic acid molecule or an oligo 23 of a predefined length, which carries the data contained in the snippet. Each nucleic acid molecule is identified by a unique identifier, index or address (e.g. contained in address segment 33 shown in Fig. 3 described below), respectively, so that the data snippets can be recombined in the right order when recovering the stored information.

The synthesized nucleic acid molecules can be stored, for example as solid matter or dissolved in a liquid, in a nucleic acid storage container, and the information can be retrieved by reading the sequences of nucleotides using a sequencer.

Biochemical storage of arbitrary digital information in DNA (Deoxyribonucleic Acid) molecules has been investigated in "Next-generation digital information storage", Church et al., Science 337, 1628, 2012 [I] and in "Towards practical, high-capacity, low-maintenance information storage in synthesized DNA", Goldman et al., Nature, 2013 [II]. Church stored about 650 kByte of data while Goldman showed that storing about 750 kByte of data is possible with available biochemical machineries. Both stored textual and media data in DNA oligos.

In Fig. 3 an example of a data carrying nucleic acid molecule or oligo 30 is illustrated. In the shown example, the DNA oligo has a total length of 159 base-pairs (bp) consisting of a 22 bp header primer segment 31, a 22 bp tail primer segment 32 and a data part consisting of a 19 bp address segment 33 and a 96 bp user data segment 34. The given lengths are merely examples. Other oligos may be composed of different amounts of base-pairs, e.g. determined by the capabilities of the synthesizer employed for synthesizing the oligos. Primers are specific sequences or series of nucleotides that are needed to process oligos biochemically, for instance to replicate them. Primers are further explained below.

In order to make the data recovering possible, for biochemical as well as technical reasons it is necessary to replicate each single information carrying oligo a certain number of times. Typically, DNA molecules are 'dissolved' in suitable chemical liquids.

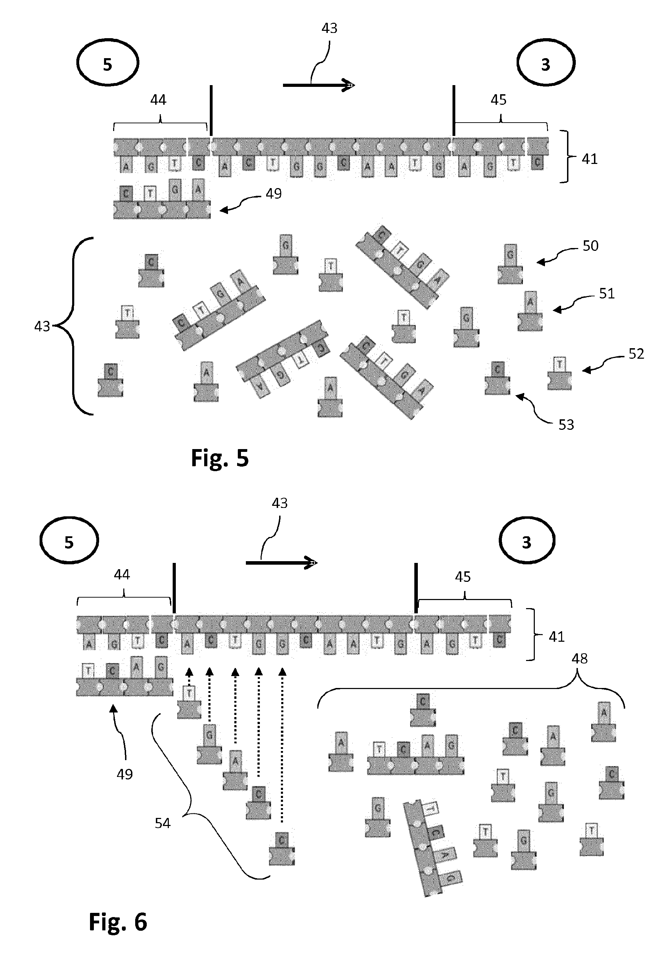

Nucleic acid molecules, i.e. oligos, can be biochemically very efficiently replicated using the Polymerase Chain Reaction (PCR). For the PCR all biochemical molecules, i.e. oligos to be replicated, unbonded primers and nucleotides, are 'poured' all together in a chemical solution. A PCR is in principle a three-step process as illustrated for DNA in Figs. 4 to 6, where like reference numerals refer to the same or similar items. The DNA replication starts at a defined series of nucleotides. These series of nucleotides are called primers. Without any loss of generality, it is assumed that primers are bonded to the beginning and end of each oligo in the solution. Primers bonded to the beginning and end of an oligo cause the whole oligo to be replicated during the PCR process. This principle was exploited by Church et al. [I] as well as Goldman [II] when carrying out their DNA storage experiments. The solution consisting of all the biochemical molecules is processed as a whole. In a first step the solution containing the oligos is heated up to a certain degree of temperature at which thermodynamic effects causes the double-stranded oligos to be separated into their two strands 41, 42, each of which comprising header and tail primer segments 44, 45, 46, 47, see Fig. 4. The arrow 43 indicates the predominant read direction (5-3). In a second step shown in Fig. 5 the heated solution 48 with the separated oligos and unbonded, i.e. not yet connected to an oligo, primers and nucleotides, which have not yet connected to suitable oligo positions, is cooled down so that some unbonded primers 49 which are provided together with single unbonded nucleotides 50, 51, 52, 53 in the solution 48, can bond at their specific positions with oligos 41 containing corresponding primer segments 44. In a third step illustrated in Fig. 6 the solution 48 is cooled down further so that the separated strands, such as the shown oligo 41, can be completed with consecutively bonding complementary nucleotides 54 so that matching base pairs can arise. At the end of the process, the oligos have been duplicated.

The primers play an important role in the PCR process; they define the starting point of the DNA replication. In order to work properly in PCRs, the series of nucleotides that constitute a primer may not match to any other series of nucleotides of any oligo in the solution.

The PCR process can be iteratively applied. The biochemical term for this process is amplification, while coverage is the biochemical term for the resulting number of replicated oligos. The amount of duplicated or replicated oligos increases exponentially with every amplification process loop. After the final PCR process there are $On = 2^n$, n = number of process loops, replications of each oligo in the solution, if all processing loops were finished without any error. However, not every PCR process circle functions perfectly. For example, Church et al. used an average coverage of about 3000 per oligo while the oligo distribution itself was in fact bell shaped.

As an example, a length of DNA oligos can be about 250 nucleotides (however, this number can be different, depending on, e.g., the capabilities of the synthesizer used for oligo generation), while primers and address indices reduce the number of data carrying base pairs to about 180. Assumed, each base pair represents 1 bit and 25 GByte of data has to be stored, then about $1.2 \times 10^9$ oligos have to be synthesized. When recovering the stored data, depending on the average oligo coverage a huge amount of oligos needs to be sequenced, for example about $1.2 \times 10^9 \times 3000 = 3.6 \times 10^{12}$. Even if only a part of the stored data has to be recovered, all the oligos have to be amplified and sequenced and decoded accordingly.

In US2005/0053968 A1 [III] it is shown that a method for storing data in DNA can make use of the primer sequences not only for enabling the PCR, but wherein consecutive segments of a data sequence are addressed by a segment number encoded in a header primer, while the tail primer is used to carry an end-of-sequence information, allowing to sort the parts of the encoded data set by means of the number encoded in the primer sequences.

If the stored data consist of multiple data sets and even if only a fragment of the stored data has to be retrieved, usually all oligos have to be amplified and sequenced and decoded accordingly, which very much slows down the access to the requested stored data.

There remains a need to provide a method and an apparatus that enables storage in and retrieval of data from synthesized nucleic acid molecules in a faster, more efficient and cost effective way.

**SUMMARY OF THE INVENTION**

A method and apparatus for storing a plurality of data sets in nucleic acid molecules, as well as a corresponding nucleic acid molecule and a method and apparatus for retrieval of selected data from such nucleic acid molecules, according to the appended claims are suggested.

According to an embodiment, a method for storing a plurality of data sets in nucleic acid molecules comprises

- partitioning the data sets into a plurality of data set portions and said data set portions into one or more data snippets; and
- synthesizing a plurality of nucleic acid molecules, at least some of which comprising
- a plurality of primer segments and
- at least one data storage segment wherein a sequence of nucleotides is arranged to encode a corresponding data snippet of a corresponding one of the data set portions of a corresponding one of the data sets, wherein
- a first primer segment comprises, i.e. has encoded data that represents, an identifier of the corresponding data set and
- a second primer segment different from the first primer segment comprises, i.e. has encoded data that represents, an identifier of the corresponding data set portion.

The term "sequence of nucleotides arranged to encode a (corresponding) data snippet" refers to a sequence of nucleotides being arranged to represent the data contained in said (corresponding) data snippet by the order of the nucleotides constituting said sequence.

The first and second primer segments belong to the plurality of primer segments. The identifier of the corresponding data set portion comprised in the second primer segment identifies a position of said data set portion the particular data snippet belongs to, within the corresponding data set, i.e. the particular data set the data set portion belongs to and which is identified using the first primer segment.

The term "at least one data storage segment" particularly refers to the situation that the data storage segment may or may not be divided into sub-segments, e.g., by additional primers not located at the beginning or end of the nucleic acid molecule.

Accordingly, an apparatus for storing a plurality of data sets in nucleic acid molecules comprises

- a partitioning unit configured to partition the data sets into a plurality of data set portions and said data set portions into one or more data snippets; and
- a synthesizer unit configured to synthesize a plurality of nucleic acid molecules, at least some of which comprising
- a plurality of primer segments and
- at least one data storage segment wherein a sequence of nucleotides is arranged to encode a corresponding data snippet of a corresponding one of the data set portions of a corresponding one of the data sets, wherein
- a first primer segment comprises an identifier of the corresponding data set and
- a second primer segment different from the first primer segment comprises an identifier of the corresponding data set portion. The synthesized nucleic acid molecules can be stored in a nucleic acid storage container for archiving and later retrieval of the encoded data.

The partitioning unit and the synthesizer unit may be provided as separate devices or jointly as one device. The partitioning unit may also be provided as functionality carried out or implemented by a microprocessor, microcontroller or other processing device, computer or other programmable apparatus connected with the synthesizer unit. The synthesizer unit may be, or be comprised in, a nucleic acid synthesizer.

According to an embodiment, a nucleic acid molecule or oligo having encoded (or carrying) a data snippet belonging to one or more data snippets of a corresponding data set portion belonging to a plurality of data set portions which belongs to a corresponding data set belonging to a plurality of data sets, comprises

- a plurality of primer segments and
- at least one data storage segment wherein a sequence of nucleotides is arranged to encode the data snippet, wherein
- a first primer segment comprises an identifier of the corresponding data set and
- a second primer segment different from the first primer segment comprises an identifier of the corresponding data set portion. A composition or solution of such nucleic acid molecules, encoding all data snippets of all data set portions of all data sets to be encoded can be stored in a nucleic acid storage container.

According to another embodiment, a method for retrieval of selected data from a plurality of nucleic acid molecules, having encoded a plurality of data sets is presented, wherein the data sets consist of a plurality of data set portions and the data set portions consist of one or more data snippets. The nucleic acid molecules comprise a plurality of primer segments and at least one data storage segment wherein a sequence of nucleotides is arranged to encode a corresponding data snippet of a corresponding one of the data set portions of a corresponding one of the data sets. A first primer segment comprises an identifier of the corresponding data set and a second primer segment different from the first primer segment comprises an identifier of the corresponding data set portion. The method comprises - amplifying, i.e. duplicating, the nucleic acid molecules using as unbonded primer segments only selected primer segments corresponding to the selected data to be retrieved. Then the amplified nucleic acid molecules are sequenced for retrieval of the selected data.

Accordingly, an apparatus for retrieval of selected data from a plurality of nucleic acid molecules, having encoded a plurality of data sets is proposed. The data sets consist of a plurality of data set portions, the data set portions consist of one or more data snippets, and the nucleic acid molecules comprise a plurality of primer segments and at least one data storage segment wherein a sequence of nucleotides is arranged to encode a corresponding data snippet of a corresponding one of the data set portions of a corresponding one of the data sets. A first primer segment comprises an identifier of the corresponding data set and a second primer segment different from the first primer segment comprises an identifier of the corresponding data set portion. The apparatus comprises - an amplification unit configured to amplify, i.e. duplicate, the nucleic acid molecules using as unbonded primer segments only selected primer segments corresponding to the data to be retrieved. Further, a sequencing unit is configured to sequence the amplified nucleic acid molecules.

The amplification unit may be a nucleic acid amplifier, such as a polymerase chain reaction (PCR) amplifier, or may be comprised in a nucleic acid sequencer.

At least in the case of more than one data snippet per data portion, the snippets may contain an index for reconstructing the sequence of data snippets. In another embodiment this index is contained in a third primer segment. In the case of each data portion consisting of only one data snippet, the second primer segment directly identifies the snippet.

In an embodiment, the snippets are assigned to nucleic acid molecules of a predefined length, i.e. consisting of a predefined amount of nucleotides, depending on the maximum length that can be synthesized by the employed synthesizer.

The provided solution allows storing of multiple sets of data, such as multiple videos, images, or other data files together in a single nucleic acid storage container and specific retrieval of only the selected data, which can be one or more selected data sets, or selected data portions of one or more or all stored data sets, as with the presented multiple primer approach a selective amplification of only the relevant stored nucleic acid molecules becomes possible, i.e. the amount of oligos that have to be amplified and afterwards sequenced in the case of data retrieval can be decreased considerably, if only a fragment of the stored data is requested. Combinations of primers can be deployed in order to access, e.g., coherently stored information or predefined sequences of all stored information. In other words, combinations of primers can be used to indicate that only selected oligos, i.e. nucleic acid molecules corresponding to a certain data set, e.g. video sequence, e.g. as a whole, or a certain data portion of one or all data sets should be replicated.

The provided approach at least has the effect that retrieval of the data becomes much more cost effective because the amplification and sequencing process can be applied much more specific and efficient. This is particularly beneficial for using nucleic acid molecules, e.g. synthesized DNA, for archival of mass data where complete amplification and following sequencing of all stored data before being able to access any probably short data item can take an unacceptably long time.

In one embodiment the synthesizing of a plurality of nucleic acid molecules comprises synthesizing Deoxyribonucleic acid (DNA) molecules. In particular for long-term archival of data the nucleic acid molecules, i.e. oligos, chosen to be synthesized are DNA molecules, as, in contrast to RNA molecules, DNA molecules are more robust to biological, biochemical and biophysical stress, like enzymes, acids and heat.

In one embodiment a modulation encoding is applied to at least one of the plurality of data sets. The modulation encoding may comprise a mapping of binary data sets of original data to quaternary data sets, as quaternary code symbols can be efficiently encoded or represented using the four nucleobases (A, T, C and G for DNA) and are, therefore, suitable for nucleic acid storage.

In one embodiment the plurality of data sets comprises at least one data set which encodes a coherent data stream. The presented approach allows access not only to selected data sets, but also to select portions within the data set to be accessed. While access to the different data sets, which can be, e.g., different files, can be realized by selecting the correct first primer, the second primer allows direct access to a specific portion of the stored file, even if it does not correspond to the beginning of a stored file. Hence, it is possible to especially access coherent data streams like videos or other large files at certain positions. These positions correspond to multiples of the size of a data portion, if the data portions have a same predefined size.

In one embodiment the plurality of primer segments comprises a third primer segment comprising a generic data set identifier, and at least some of the nucleic acid molecules are synthesized using said third primer segment. A generic data set identifier is an identifier dedicated to identifying a common feature of certain data sets. A generic data set identifier may, for example, identify stored movies (while the first primer identifies the specific movie), while another generic data set identifier may identify images or text files. This enables request for retrieving, for example, all movies stored in a nucleic acid storage which also contains other stored data.

In one embodiment the plurality of primer segments comprises a fourth primer segment comprising a generic data set portion identifier. The generic data set portion identifier identifies the type of data contained in a data set portion. For example, if the data set is a movie, the generic data set portion identifier contained in the fourth primer indicates that the second primer identifies specific sequences within the movie, for example, specific sequences of a uniform duration.

In one embodiment the plurality of primer segments comprises at least one additional primer segment encoding additional metadata of the corresponding data set. This allows identification of the stored data for selective retrieval according to any other metadata classifying the encoded content. The additional primer segment may, for example, have encoded metadata for identifying a specific owner of the data, the genre of encoded movies or other information classifying the encoded content.

Two sequences of nucleotides are considered "reverse complementary" to each other, if an antiparallel alignment of the nucleotide sequences results in the nucleobases at each position being complementary to their counterparts. Reverse complementarity does not only occur between separate sequences. It is also possible for a sequence or section of nucleotides to have internal or self-reverse complementarity. For example, for an oligo section $o(i: j) = (n_i, n_{i+1} ..., n_j)$,

$0 \leq i \leq j \leq N - 1$ the reverse complementary oligo section is given by $\overline{o(i:j)} = \left( \overline{n_j}, \overline{n_{j-1}}, ..., \overline{n_i} \right)$, $N - 1 \geq j \geq i \geq 0$. For

example, for a self reverse complementary oligo, the following equation applies: $o = \overline{o} = \widetilde{\overline{o}}.$

A self-reverse complementary section or stretch within an oligo may result in a folded configuration where the sequence binds to itself and may, for example, form "hairpins" or loops, which may not always be correctly sequenced. Therefore, in one embodiment the plurality of primer segments does not comprise a primer segment that exhibits by itself or concatenated with another of the primer segments self-reverse complementarity.

For selective data retrieval, in one embodiment the amplifying comprises using as the unbonded primer segments first primer segments which comprise an identifier of at least one selected data set but no second primer segments which comprise an identifier of a data set portion. Providing as unbonded primer segments only first primers identifying a data set allows efficient selective amplification of oligos corresponding to a complete data set, for example a selected file, as a whole.

In another embodiment the amplifying comprises using as the unbonded primer segments second primer segments which comprise an identifier of at least one selected data set portion but no first primer segments which comprise an identifier of a data set. This allows easy amplification of selected oligos corresponding to a selected data set portion within multiple data sets, for example the first, introductory data set portion of each data set in a nucleic acid storage.

In one embodiment the amplifying comprises using as some of the unbonded primer segments third primer segments comprising a generic data set identifier. This enables selective amplification of oligos and later retrieval of corresponding multiple data sets identified by the generic data set identifier, for example all movie files in a nucleic acid storage containing diverse data sets, such as movies, images, text files etc. In one embodiment the amplifying comprises using as some of the unbonded primer segments fourth primer segments comprising a generic data set portion identifier.

While not explicitly described, the presented embodiments may be employed in any combination or sub-combination.

## BRIEF DESCRIPTION OF THE DRAWINGS

Fig. 1    schematically illustrates a structure of a fragment of a DNA molecule;

Fig. 2    schematically illustrates a principle of data assignment to oligos to be used for data storage;

Fig. 3    schematically illustrates an example of a prior art data carrying oligo;

Fig. 4    schematically illustrates a first step of a principle of DNA replication by polymerase chain reaction - separation of DNA strands;

Fig. 5    schematically illustrates a second step of a principle of DNA replication by polymerase chain reaction - primers of the solution bond to the primers of the separated strands;

Fig. 6     schematically illustrates a third step of a principle of DNA replication by polymerase chain reaction - nucleotides of the solution bond to their corresponding complementary nucleotides forming base pairs;

Fig. 7     schematically illustrates an embodiment of a method for storing a plurality of data sets in nucleic acid molecules;

Fig. 8     schematically illustrates an embodiment of an apparatus for storing a plurality of data sets in nucleic acid molecules;

Fig. 9     schematically illustrates an embodiment of a method for retrieval of selected data from a plurality of nucleic acid molecules having encoded a plurality of data sets;

Fig. 10    schematically illustrates an embodiment of an apparatus for retrieval of selected data from a plurality of nucleic acid molecules having encoded a plurality of data sets;

Fig. 11    schematically illustrates an example of storage of several movies in a nucleic acid solution;

Fig. 12    schematically illustrates an example of a primer and its inverse;

Fig. 13    schematically illustrates an example of a self-reverse complementary primer;

Fig. 14    schematically illustrates an example of a movie data snippet stored in a first embodiment of a nucleic acid molecule; and

Fig. 15    schematically illustrates an example of a movie data snippet stored in a second embodiment of a nucleic acid molecule.

## DETAILED DESCRIPTION OF EMBODIMENTS

For a better understanding, the proposed solution will now be explained in more detail in the following description with reference to the drawings. It is understood that the solution is not limited to these exemplary embodiments and that specified features can also expediently be combined and/or modified without departing from the scope of the present invention as defined in the appended claims.

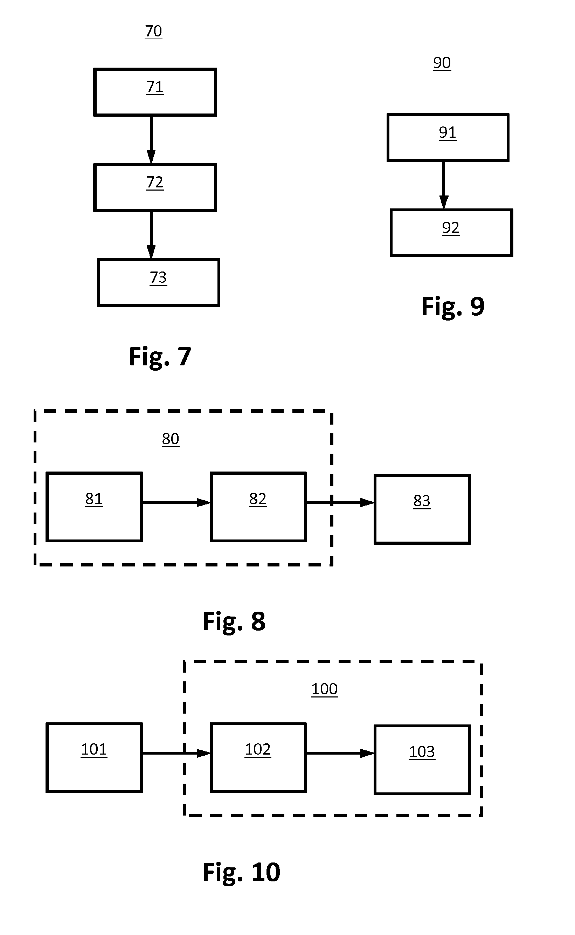

Referring to Fig. 7, an embodiment of a method 70 for storing a plurality of data sets in nucleic acid molecules, e.g. synthesized DNA oligos, is schematically shown. Apart from the synthesizing of the nucleic acid molecules using, e.g., a synthesizer, the method may, for example, be computer implemented.

In the shown embodiment, in a first step 71 a modulation encoding is applied to at least one of the plurality of data sets.

In a second step 72 the data sets are then partitioned into a plurality of data set portions and said data set portions are partitioned into one or more data snippets.

In a third step 73 a plurality of nucleic acid molecules is synthesized such that least some of the synthesized nucleic acid molecules comprise

- a plurality of primer segments and
- at least one data storage segment wherein a sequence of nucleotides is arranged to encode a corresponding data snippet of a corresponding one of the data set portions of a corresponding one of the data sets, wherein
- a first primer segment comprises an identifier of the corresponding data set and
- a second primer segment different from the first primer segment comprises an identifier of the corresponding data set portion.

Accordingly, an embodiment of an apparatus 80 for storing a plurality of data sets in nucleic acid molecules is schematically shown in Fig. 8. The apparatus 80 allows implementing the advantages and characteristics of the described method as part of an apparatus for storing a plurality of data sets in nucleic acid molecules.

The apparatus 80 comprises a partitioning unit 81 configured to partition the data sets into a plurality of data set portions and said data set portions into one or more data snippets.

The partitioning unit 81 is connected to a synthesizer unit 82 configured to synthesize a plurality of nucleic acid molecules, at least some of which comprising

- a plurality of primer segments and
- at least one data storage segment wherein a sequence of nucleotides is arranged to encode a corresponding data snippet of a corresponding one of the data set portions of a corresponding one of the data sets, wherein
- a first primer segment comprises an identifier of the corresponding data set and
- a second primer segment different from the first primer segment comprises an identifier of the corresponding data set portion.

The partitioning unit 81 and the synthesizer unit 82 comprised in the embodiment of the apparatus 80 shown in Fig. 8 may be provided as separate devices or jointly as one device. The partitioning unit 81 may be provided as functionality carried out or implemented by a microprocessor, microcontroller or other processing device, computer or other programmable apparatus.

In the shown embodiment the synthesizer unit 82 is connected to a nucleic acid storage 83 configured to store the plurality of nucleic acid molecules synthesized by the synthesizer unit 82. In another embodiment the nucleic acid storage is comprised in the apparatus 80, for example as part of the synthesizer unit 82.

Referring now to Fig. 9, an embodiment of a method 90 for retrieval of selected data from a plurality of nucleic acid molecules having encoded a plurality of data sets is schematically shown. Herein, the data sets consist of a plurality of data set portions, the data set portions consist of one or more data snippets, and the nucleic acid molecules comprise a plurality of primer segments and at least one data storage segment wherein a sequence of nucleotides is arranged to encode a corresponding data snippet of a corresponding one of the data set portions of a corresponding one of the data sets, wherein a first primer segment comprises an identifier of the corresponding data set and a second primer segment different from the first primer segment comprises an identifier of the corresponding data set portion.

The method 90 comprises a step 91 of amplifying the nucleic acid molecules using as unbonded primer segments only selected primer segments corresponding to the data to be retrieved.

In the shown embodiment the method 90 comprises a further step 92 wherein the amplified nucleic acid molecules are sequenced for retrieval of the selected data.

Accordingly, an embodiment of an apparatus 100 for retrieval of selected data from a plurality of nucleic acid molecules having encoded a plurality of data sets is schematically illustrated in Fig. 10. The apparatus 100 allows implementing the advantages and characteristics of the described method as part of an apparatus for retrieval of selected data from a plurality of nucleic acid molecules having encoded a plurality of data sets.

Herein, the data sets consist of a plurality of data set portions, the data set portions consist of one or more data snippets, and the nucleic acid molecules comprise a plurality of primer segments and at least one data storage segment wherein a sequence of nucleotides is arranged to encode a corresponding data snippet of a corresponding one of the data set portions of a corresponding one of the data sets, wherein a first primer segment comprises an identifier of the corresponding data set and a second primer segment different from the first primer segment comprises an identifier of the corresponding data set portion.

In the shown embodiment the apparatus 100 is connected to receive these nucleic acid molecules from a nucleic acid storage 101. In another embodiment the nucleic acid storage 101 is part of the apparatus 100.

The apparatus 100 comprises an amplification unit 102 configured to amplify the nucleic acid molecules using as unbonded primer segments only selected primer segments corresponding to the data to be retrieved. In the shown embodiment the apparatus 100 further comprises a sequencing unit 103 configured to sequence the amplified nucleic acid molecules.

In an embodiment a nucleic acid storage system is provided which comprises apparatus 80 for storing a plurality of data sets in nucleic acid molecules, apparatus 100 for retrieval of selected data from a plurality of nucleic acid molecules having encoded a plurality of data sets and the nucleic acid storage 101 which is identical to the nucleic acid storage 83 shown in Fig. 8.

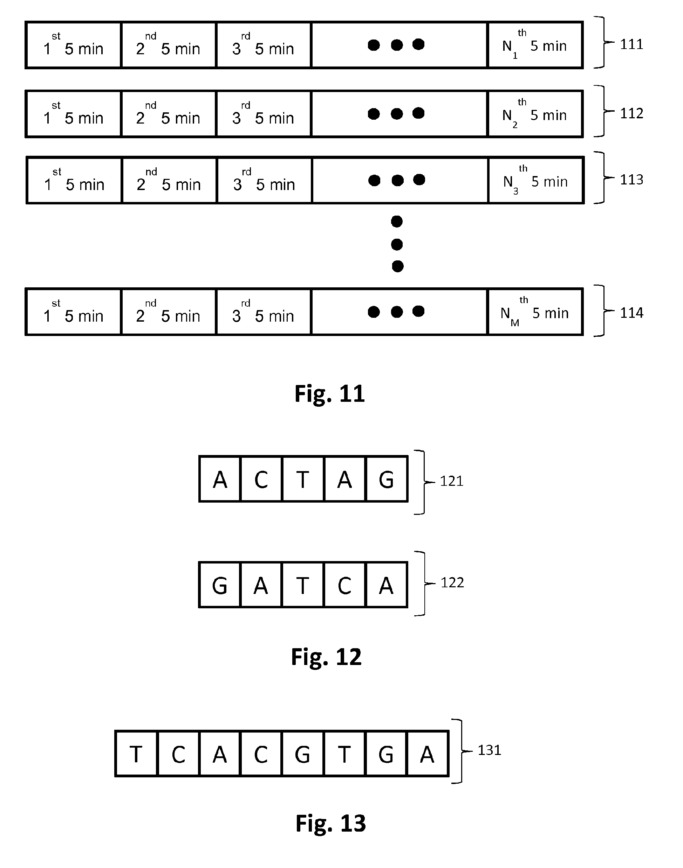

Referring now to Figs. 11 to 15, as an example, storage of multiple movies in DNA molecules, i.e. oligos, is described, wherein the need to sequence the complete amount of storage oligos is overcome, if only a subset of the oligos is carrying the data that has to be recovered, such that the amount of oligos that have to be sequenced can be decreased considerably. As described above, concatenating dedicated primers to the beginnings and ends of the oligos enables selective amplification of oligos using PCR biochemical processes.

In Fig. 11 an example of storage of several movies in a single DNA solution is schematically illustrated, wherein it is assumed that a first movie 111, a second movie 112, a third movie 113, ... an $m^{th}$ movie 114 to be stored in DNA are divided into $N_1$, $N_2$, $N_3$, ... $N_m$ 5 minutes sequences, respectively. The amounts $N_1$, $N_2$, $N_3$, ... $N_m$ of 5 minutes sequences can be identical or different from each other.

When carrying out a PCR process, all oligos are amplified whose primers match the applied PCR primer. The primers as well as concatenations of the primers may not be self-reverse complementary. For primer-generation this constraint is taken into account. Fig. 12 schematically illustrates an example of a primer $PS_n$ 121 and its reverse or inverse $^{inv}PS_n$ 122. Fig. 13 schematically illustrates an example of a self-reverse-complementary primer 131. Further, primers may not match to any other series of nucleotides of any of the oligos in the PCR solution.

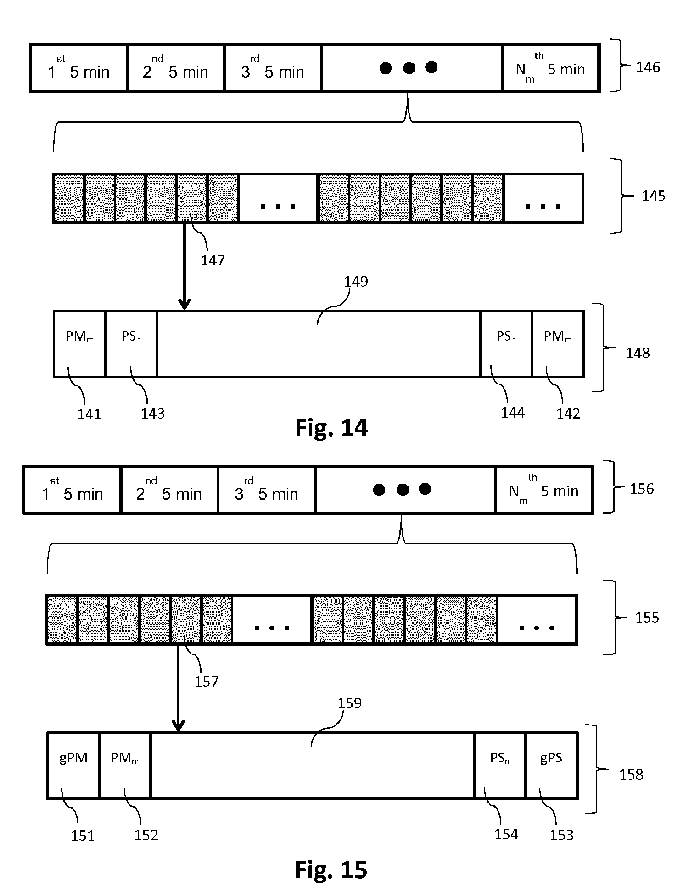

Referring to Fig. 14, an example of a movie data snippet stored in a first embodiment of a nucleic acid molecule, here a DNA oligo, is schematically illustrated. Here, two sorts of primers are assigned to the movie data carrying oligos. The first primer (PM) indicates the data set, i.e. the stored movie, while the data set portions, i.e. the consecutive 5 minutes sequences, are individually labeled with primers out of a set of secondary primers (PS).

Fig. 14 shows exemplary how the corresponding primers $PM_m$ 141, 142 and primers $PS_n$ 143, 144 are assigned to the $n^{th}$ 5 minutes sequence 145 of the $m^{th}$ stored movie 146. The data of the $n^{th}$ 5 minutes sequence 145 is divided into fractions of data, i.e. data snippets. In the shown example a snippet 147 of the data of the $n^{th}$ 5 minutes sequence 145 is assigned to an oligo 148, i.e. the oligo 148 is synthesized, having a data storage segment 149 carrying the data of the data snippet 147 and having a primer series $PM_m+PS_n$ assigned to its beginning and end.

The set of primers PM for M movies is:

$$\{PM_1, PM_2, PM_3, ..., PM_m, ... PM_M\}.$$

The set of primers PS for all 5 minutes sequences is:

$\{PS_1, PS_2, PS_3, ... PS_n, ... , PS_N\}$, while $N_1, N_2, N_3, N_m, N_m \leq N$, wherein the different indices $N_m$ of the of 5 minutes sequence primers PS refer to movies that are generally different in length.

The set of primers PM labels unambiguously the stored movies in the DNA solution as shown in Table 1:

Table 1

| Primers PM | Movie |
|---|---|
| $PM_1$ | Movie 1 |
| $PM_2$ | Movie 2 |
| ... | ... |
| $PM_m$ | Movie m |
| ... | ... |
| $PM_M$ | Movie M |

The set of primers PS labels unambiguously the consecutive 5 minutes sequences of the stored movies, as shown in Table 2:

Table 2

| Primers PS | 5 minute sequences |
|---|---|
| $PS_1$ | Sequence 1 |
| $PS_2$ | Sequence 2 |
| ... | ... |
| $PS_n$ | Sequence n |
| ... | ... |
| $PS_N$ | Sequence N |
| $N_1, N_2, N_3, N_m, N_m \leq N$ $N_m$ indicates a 5 min. sequence of movie m | |

If, for example, the $n^{th}$ 5 minutes sequence of the $m^{th}$ movie has to be recovered, a series of PCR amplification processes is carried out deploying the series of primers $PM_m+PS_n$ labelling the requested $n^{th}$ 5 minute sequence of the $m^{th}$ movie. The mechanism is based on the fact that the PCR process amplifies only those oligos that have the corresponding labelling primer attached to its beginning and end.

A dedicated primer construction rule is deployed which ensures that the series of nucleotides that constitute a primer does not match to any other series of nucleotides of any of the oligos in the PCR solution. The amount of oligos that have to be sequenced is decreased considerably, if only some of the stored data is requested, for example in the described case where several movies are stored together in one DNA solution of oligos and only parts of the data have to be recovered.

The following combinations of the dedicated primers PM and PS select the corresponding data as shown in Table 3:

Table 3

| Applied Primers | Selected data |
|---|---|
| $PM_m$ | $m^{th}$ movie |
| $PM_m + PS_n$ | $n^{th}$ 5 minute sequence of the $m^{th}$ movie |
| $1 \leq m \leq M$ and $1 \leq n \leq N_m$ <br> M movies, $N_m$ indicates a 5 minutes sequences of movie m | |

Applying primer $PM_m$ 141, 142 causes the whole $m^{th}$ movie 146 to be amplified. Applying primer combination $PM_m + PS_n$ 141, 143; 142, 144 causes the $n^{th}$ 5 minute sequence 145 of the $m^{th}$ movie 146 to be selectively amplified.

Referring now to Fig. 15, an example of a movie data snippet stored in a second embodiment of a nucleic acid molecule, here a DNA oligo, is schematically illustrated. Here, the primers concatenated to the oligos vary, thus making further selective data recovering modes possible. At the beginning of each oligo, a generic primer (gPM) indicating movies is concatenated with primers indicating individual movies ($PM_m$). At the end of each oligo, primers ($PS_n$) indicating individual single 5 minutes sequences is concatenated with a generic primer (gPS) indicating sequences.

Fig. 15 shows exemplary the alignment of oligos and primers, in particular the primer concatenation $gPM + PM_m$ as well as the concatenation of $PS_n + gPS$ assigned to the $n^{th}$ 5 minutes sequence of the $m^{th}$ movie, i.e. how the generic primer gPM 151 indicating movies, the primer $PM_m$ 152 indicating an individual movie, the generic primer 153 indicating sequences and the primer $PS_n$ 154 indicating an individual sequence are assigned to the $n^{th}$ 5 minutes sequence 155 of the $m^{th}$ stored movie 156. The data of the $n^{th}$ 5 minutes sequence 155 is divided into fractions of data, i.e. data snippets. In the shown example a snippet 157 of the data of the $n^{th}$ 5 minutes sequence 155 is assigned to an oligo 158, i.e. the oligo 158 is synthesized, having a data storage segment 159 carrying the data of the data snippet 157 and having the primer series $gPM + PM_m$ 151, 152 assigned to its beginning and the primer series $PS_n + gPS$ 154, 153 assigned to its end.

The set of primers gPM and $PM_m$ for M movies is:

$$\{gPM, PM_1, PM_2, PM_3, ..., PM_m, ... PM_M\}$$

The set of primers $PS_n$ and gPS for all 5 minutes sequences is:

$$\{PS_1, PS_2, PS_3, ... PS_n, ..., PS_N, gPS\}, \text{ while } N_1, N_2, N_3, N_m, N_m \leq N$$

The different indices $N_m$ of the 5 minutes sequence primers PS refer to movies that are generally different in length. The described mechanism allows, for example, to have all the oligos carrying the $n^{th}$ 5 minute sequences of all stored movies amplified by PCR processes, and thus all the data of all 5 minutes sequences of all stored movies can be recovered at once.

In the following Table 4 the set of concatenated primers gPM+PM are listed labelling unambiguously the stored movies in the DNA solution:

Table 4

| Concatenated Primers gPM+PM | Movie |
|---|---|
| $gPM + PM_1$ | Movie 1 |
| $gPM + PM_2$ | Movie 2 |
| ... | ... |
| $gPM + PM_m$ | Movie m |
| ... | ... |
| $gPM + PM_M$ | Movie M |

In Table 5 the set of concatenated primers PS+gPS is listed labelling unambiguously the consecutive sequences of 5 minutes of the stored movies:

Table 5

| Concatenated Primers PS+gPS | 5 minute sequences |
|---|---|
| $PS_1$+gPS | Sequence 1 |
| $PS_2$+gPS | Sequence 2 |
| ... | ... |
| $PS_n$+gPS | Sequence n |
| ... | ... |
| $PS_N$+gPS | Sequence N |
| $N_1$, $N_2$, $N_3$, $N_m$, $N_m \leq N$<br>$N_m$ 5 minutes sequences of movie m | |

Table 6 shows primer combinations and the correspondingly selected stored data. The combinations of the concatenated primers gPM+PM and PS+gPS makes it possible to selectively recover data as listed in Table 6:

Table 6

| Applied Primers | Selected data |
|---|---|
| gPM+$PM_m$<br>and<br>gPS | $m^{th}$ movie |
| $PS_n$+gPS<br>and<br>gPM | $n^{th}$ 5 minute sequence of all M movies |
| $1 \leq m \leq M$ and $1 \leq n \leq N_m$<br>M movies, $N_m$ 5 minutes sequences of movie m | |

Applying the primer concatenation gPM+$PM_m$ 151, 152 together with the generic primer gPS 153 causes the whole $m^{th}$ movie 156 to be amplified. Differently to the first embodiment shown in Fig. 14, for a DNA oligo shown in Fig. 15 only one strand of the corresponding oligo 158 reacts to a primer concatenation gPM+$PM_m$ 151, 152 while the other strand reacts to a generic sequence primer gPS 153. As a result, the demanded oligos are amplified.

Applying the primer concatenation $PS_n$+gPS 154, 153 together with the generic movie primer gPM 151 allows selective amplification and recovery of all $n^{th}$ 5 minute sequence of all M movies. Differently to the first embodiment shown in Fig. 14, for a DNA oligo shown in Fig. 15 only one strand of the corresponding oligos reacts to a primer concatenation $PS_n$+gPS 154, 153 while the other strand reacts to a generic movie primer gPM 151. As a result, the demanded oligos are amplified.

In both cases, a dedicated primer construction rule is deployed ensuring that the series of nucleotides that constitute a primer does not match to any other series of nucleotides of any of the oligos in the PCR solution.

Aspects of the present principles can take the form of a hardware embodiment, a software embodiment or an embodiment combining software and hardware aspects. Aspects of the present principles may, for example, at least partly be implemented in a computer program comprising code portions for performing steps of the method according to an embodiment of the invention when run on a programmable apparatus or enabling a programmable apparatus to perform functions of an apparatus or system according to an embodiment of the invention.

**CITATION LIST**

**Non-patent literature**

[I] George M. Church, Yuan Gao, Sriram Kosuri, "Next-Generation Digital Information Storage in DNA", Science Vol. 337, 28 September 2012.

[II] Nick Goldman et al., "Towards practical, high-capacity, low-maintenance information storage in synthesized DNA", Nature LETTER, Nature11875, Nature, January 2013.

**Patent literature**

[III] US 2005/0053968 A1

**Claims**

1.  Method (70) for storing a plurality of data sets in nucleic acid molecules, comprising

    - partitioning (72) the data sets into a plurality of data set portions and said data set portions into one or more data snippets; and
    - synthesizing (73) a plurality of nucleic acid molecules, at least some of which comprising

        - a plurality of primer segments and
        - at least one data storage segment wherein a sequence of nucleotides is arranged to encode a corresponding data snippet of a corresponding one of the data set portions of a corresponding one of the data sets, wherein
        - a first primer segment comprises an identifier of the corresponding data set and
        - a second primer segment different from the first primer segment comprises an identifier of the corresponding data set portion.

2.  Method according to claim 1, wherein the synthesizing (73) of a plurality of nucleic acid molecules comprises synthesizing Deoxyribonucleic acid molecules.

3.  Method according to claim 1 or claim 2, comprising applying(71) a modulation encoding to at least one of the plurality of data sets.

4.  Method according to any of the preceding claims, wherein the plurality of data sets comprises at least one data set which encodes a coherent data stream.

5.  Method according to any of the preceding claims, wherein

    - the plurality of primer segments comprises a third primer segment comprising a generic data set identifier, and
    - at least some of the nucleic acid molecules are synthesized using said third primer segment.

6.  Method according to any of the preceding claims, wherein the plurality of primer segments comprises a fourth primer segment comprising a generic data set portion identifier.

7.  Method according to any of the preceding claims, wherein the plurality of primer segments comprises at least one additional primer segment encoding additional metadata of the corresponding data set.

8.  Method according to any of the preceding claims, wherein the plurality of primer segments does not comprise a primer segment that exhibits by itself or concatenated with another of the primer segments self-reverse complementarity.

9.  Method (90) for retrieval of selected data from a plurality of nucleic acid molecules having encoded a plurality of data sets, the data sets consisting of a plurality of data set portions, the data set portions consisting of one or more data snippets, the nucleic acid molecules comprising a plurality of primer segments and at least one data storage segment wherein a sequence of nucleotides is arranged to encode a corresponding data snippet of a corresponding one of the data set portions of a corresponding one of the data sets, wherein a first primer segment comprises an identifier of the corresponding data set and a second primer segment different from the first primer segment comprises an identifier of the corresponding data set portion, the method comprising

- amplifying (91) the nucleic acid molecules using as unbonded primer segments only selected primer segments corresponding to the selected data to be retrieved.

10. Method according to claim 9, wherein the amplifying comprises using as the unbonded primer segments first primer segments which comprise an identifier of at least one selected data set but no second primer segments which comprise an identifier of a data set portion.

11. Method according to claim 9, wherein the amplifying comprises using as the unbonded primer segments second primer segments which comprise an identifier of at least one selected data set portion but no first primer segments which comprise an identifier of a data set.

12. Method according to any of claims 9 to 11, wherein the amplifying comprises using as some of the unbonded primer segments third primer segments comprising a generic data set identifier.

13. Nucleic acid molecule (148) having encoded a data snippet (147) belonging to one or more data snippets of a corresponding data set portion (145) belonging to a plurality of data set portions which belongs to a corresponding data set (146) belonging to a plurality of data sets, the nucleic acid molecule comprising

- a plurality of primer segments (141, 142, 143, 144) and
- at least one data storage segment (149) wherein a sequence of nucleotides is arranged to encode the data snippet (147), wherein
- a first primer segment (141, 142) comprises an identifier of the corresponding data set (146) and
- a second primer segment (143, 144) different from the first primer segment (141,142) comprises an identifier of the corresponding data set portion (145).

14. Apparatus (80) for storing a plurality of data sets in nucleic acid molecules, comprising

- a partitioning unit (81) configured to partition the data sets into a plurality of data set portions and said data set portions into one or more data snippets; and
- a synthesizer unit (82) configured to synthesize a plurality of nucleic acid molecules, at least some of which comprising

- a plurality of primer segments and
- at least one data storage segment wherein a sequence of nucleotides is arranged to encode a corresponding data snippet of a corresponding one of the data set portions of a corresponding one of the data sets, wherein
- a first primer segment comprises an identifier of the corresponding data set and
- a second primer segment different from the first primer segment comprises an identifier of the corresponding data set portion.

15. Apparatus (100) for retrieval of selected data from a plurality of nucleic acid molecules having encoded a plurality of data sets, the data sets consisting of a plurality of data set portions, the data set portions consisting of one or more data snippets, the nucleic acid molecules comprising a plurality of primer segments and at least one data storage segment wherein a sequence of nucleotides is arranged to encode a corresponding data snippet of a corresponding one of the data set portions of a corresponding one of the data sets, wherein a first primer segment comprises an identifier of the corresponding data set and a second primer segment different from the first primer segment comprises an identifier of the corresponding data set portion, the apparatus comprising

- an amplification unit (102) configured to amplify the nucleic acid molecules using as unbonded primer segments only selected primer segments corresponding to the selected data to be retrieved.

Fig. 1

Fig. 2

**Fig. 3**

**Fig. 4**

**Fig. 5**

**Fig. 6**

70

71

72

73

**Fig. 7**

90

91

92

**Fig. 9**

80

81

82

83

**Fig. 8**

100

101

102

103

**Fig. 10**

| $1^{st}$ 5 min | $2^{nd}$ 5 min | $3^{rd}$ 5 min | • • • | $N_1^{th}$ 5 min |
|---|---|---|---|---|

⎫ 111

| $1^{st}$ 5 min | $2^{nd}$ 5 min | $3^{rd}$ 5 min | • • • | $N_2^{th}$ 5 min |
|---|---|---|---|---|

⎫ 112

| $1^{st}$ 5 min | $2^{nd}$ 5 min | $3^{rd}$ 5 min | • • • | $N_3^{th}$ 5 min |
|---|---|---|---|---|

⎫ 113

•
•
•

| $1^{st}$ 5 min | $2^{nd}$ 5 min | $3^{rd}$ 5 min | • • • | $N_M^{th}$ 5 min |
|---|---|---|---|---|

⎫ 114

**Fig. 11**

| A | C | T | A | G |
|---|---|---|---|---|

⎫ 121

| G | A | T | C | A |
|---|---|---|---|---|

⎫ 122

**Fig. 12**

| T | C | A | C | G | T | G | A |
|---|---|---|---|---|---|---|---|

⎫ 131

**Fig. 13**

| 1$^{st}$ 5 min | 2$^{nd}$ 5 min | 3$^{rd}$ 5 min | ● ● ● | N$_m$$^{th}$ 5 min | 146 |

145

147

| PM$_m$ | PS$_n$ | 149 | | PS$_n$ | PM$_m$ | 148 |

141    143

**Fig. 14**

| 1$^{st}$ 5 min | 2$^{nd}$ 5 min | 3$^{rd}$ 5 min | ● ● ● | N$_m$$^{th}$ 5 min | 156 |

155

157

| gPM | PM$_m$ | 159 | | PS$_n$ | gPS | 158 |

151    152

**Fig. 15**

**Europäisches Patentamt**
**European Patent Office**
**Office européen des brevets**

# EUROPEAN SEARCH REPORT

Application Number

EP 15 30 5381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MASAHITO YAMAMOTO ET AL: "Large-scale DNA memory based on the nested PCR", NATURAL COMPUTING, KLUWER ACADEMIC PUBLISHERS, DO, vol. 7, no. 3, 19 March 2008 (2008-03-19), pages 335-346, XP019612124, ISSN: 1572-9796 * The whole document, e.g. Figs. 1, 2 * | 1-15 | INV. G06F19/10 G06N3/12 G11C13/00 |
| A,D | CHURCH ET AL.: "Next-generation digital information storage", SCIENCE, vol. 337, 2012, page 1628, XP055082578, * the whole document * | 1-15 | |
| A,D | NICK GOLDMAN ET AL.: "Towards practical, high-capacity, low-maintenance information storage in synthesized DNA", NATURE LETTER, NATURE11875, NATURE, January 2013 (2013-01), XP055050963, * the whole document * | 1-15 | |

| | |
|---|---|
| | TECHNICAL FIELDS SEARCHED (IPC) |
| | G06F G06N G11C |

The present search report has been drawn up for all claims

| Place of search | Date of completion of the search | Examiner |
|---|---|---|
| Munich | 8 September 2015 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050053968 A1

**Non-patent literature cited in the description**

- **CHURCH et al.** Biochemical storage of arbitrary digital information in DNA (Deoxyribonucleic Acid) molecules has been investigated in ''Next-generation digital information storage. *Science,* 2012, vol. 337, 1628
- **GOLDMAN et al.** Towards practical, high-capacity, low-maintenance information storage in synthesized DNA. *Nature,* 2013
- **GEORGE M. CHURCH ; YUAN GAO ; SRIRAM KOSURI.** Next-Generation Digital Information Storage in DNA. *Science,* 28 September 2012, vol. 337
- **NICK GOLDMAN et al.** Towards practical, high-capacity, low-maintenance information storage in synthesized DNA. *Nature LETTER, Nature11875, Nature,* January 2013